# EUROPEAN PATENT SPECIFICATION

(11) **EP 2 138 091 B1**
(45) Date of publication and mention of the grant of the patent: **19.06.2013**
(21) Application number: 07742295.4
(22) Date of filing: 24.04.2007
(51) Int. Cl.: G06T 7/00, A61B 1/04, A61B 6/00, A61B 6/03, G02B 23/24

(54) **MEDICAL IMAGE PROCESSING APPARATUS AND MEDICAL IMAGE PROCESSING METHOD**
MEDIZINISCHE BILDVERARBEITUNGSVORRICHTUNG UND MEDIZINISCHES BILDVERARBEITUNGSVERFAHREN
DISPOSITIF DE TRAITEMENT D'IMAGES MÉDICALES ET PROCÉDÉ DE TRAITEMENT D'IMAGES MÉDICALES

(43) Date of publication of application: 30.12.2009
(73) Proprietor: Olympus Medical Systems Corp., Tokyo 151-0072 (JP)
(72) Inventor: INOUE, Ryoko, Tokyo 151-0072 (JP)
(74) Representative: von Hellfeld, Axel
(86) International application number: PCT/JP2007/058860
(87) International publication number: WO 2008/136098

(56) References cited:
- JP-A- 10 014 864
- JP-A- 2000 155 840
- JP-A- 2002 165 757
- JP-A- 2005 157 902
- JP-A- 2005 192 880
- JP-A- 2006 166 939
- JP-A- 2006 166 990
- US-A1- 2003 223 627
- ZHANG R ET AL: "SHAPE FROM SHADING: A SURVEY", IEEE TRANSACTIONS ON PATTERN ANALYSIS AND MACHINE INTELLIGENCE, IEEE SERVICE CENTER, LOS ALAMITOS, CA, US, vol. 21, no. 8, 1 August 1999 (1999-08-01) , pages 690-706, XP008057369, ISSN: 0162-8828, DOI: 10.1109/34.784284
- DUROU ET AL: "Numerical methods for shape-from-shading: A new survey with benchmarks", COMPUTER VISION AND IMAGE UNDERSTANDING, ACADEMIC PRESS, US, vol. 109, no. 1, 14 December 2007 (2007-12-14), pages 22-43, XP022391253, ISSN: 1077-3142, DOI: 10.1016/J.CVIU.2007.09.003
- KIMURA T.: 'Keijo Joho ni Motozuku 3-Jigen Fukubu CT-zo kara no Daicho Polyp Jido Kenshutsu Shuho ni Kansuru Kento.' IEICE TECHNICAL REPORT 2004, pages 29 - 34, XP003020670

## Description

### Technical Field

The present invention relates to a medical image processing apparatus and a medical image processing method and, more particularly, to a medical image processing apparatus and a medical image processing method for estimating a three-dimensional model of a living body tissue on the basis of a two-dimensional image of the living body tissue.

### Background Art

Observation using a piece of image pickup equipment such as an X-ray diagnosis apparatus, CT, MRI, ultrasound observation apparatus, or endoscope apparatus has been prevalent in a field of medicine. Of such pieces of image pickup equipment, an endoscope apparatus has, e.g., a following function and configuration: the endoscope apparatus has an insertion portion capable of being inserted into a body cavity, and the endoscope apparatus picks up an image of an inside of a body cavity formed by an objective optical system arranged at a distal end portion of the insertion portion using image pickup means such as a solid-state image pickup device and outputs the image as an image pickup signal and displays an image of the inside of the body cavity on display means such as a monitor on the basis of the image pickup signal. A user observes, e.g., an organ in a body cavity on the basis of an image of an inside of the body cavity displayed on the display means such as a monitor.

An endoscope apparatus is capable of directly picking up an image of a digestive tract mucous membrane. Accordingly, a user can comprehensively observe, e.g., a color of a mucous membrane, a shape of a lesion, a fine structure on a surface of the mucous membrane, and the like. Endoscope apparatuses capable of estimating a three-dimensional model of a picked-up image of an inside of a body cavity on the basis of two-dimensional image data corresponding to the image of the inside of the body cavity have been proposed in recent years.

An endoscope apparatus can also detect an image including a lesioned part such as a polyp by using, e.g., an image processing method described in Japanese Patent Application Laid-Open Publication No. 2005-192880 as an image processing method capable of detecting a predetermined image in which a lesion with a locally raised shape is present.

A two-dimensional image including, e.g., an image of a living body tissue such as a fold of a large intestine or a polyp as an image of a living body tissue with a raised shape picked up by an endoscope often includes an invisible region with respect to a direction of view of the endoscope. The invisible region in the two-dimensional image is commonly referred to as occlusion.

The image processing method in Japanese Patent Application Laid-Open Publication No. 2005-192880 has a problem in that estimation based on three-dimensional data with low reliability present in occlusion described above causes a reduction in an efficiency of detecting a raised shape, for example, if the image processing method is used as a process for estimating a three-dimensional model on the basis of a two-dimensional image acquired by an endoscope.

The present invention has been made in consideration of the above-described points, and has as an object to provide a medical image processing apparatus and a medical image processing method capable of making an efficiency of detecting a raised shape higher than ever before when a three-dimensional model is estimated on the basis of a two-dimensional image acquired by an endoscope.

### Disclosure of Invention

### Means for Solving the Problem

Some or all of the above problems are overcome by the medical image processing apparatuses according of claims 1 and 3, and the medical image processing methods according to claims 6 and 8.

A medical image processing apparatus of a first aspect according to the present invention includes an edge extraction portion that extracts an edge in a two-dimensional image of a living body tissue inputted from a medical image pickup apparatus, based on the two-dimensional image, a three-dimensional model estimation portion that estimates a three-dimensional model of the living body tissue, based on the two-dimensional image, a local region setting portion that sets a local region centered on a pixel of interest in the two-dimensional image, a determination portion that determines whether the local region is divided by at least part of the edge extracted by the edge extraction portion, a shape feature value calculation portion that calculates a shape feature value of the pixel of interest using three-dimensional coordinate data corresponding to, of the local region, a region which is not divided by the edge extracted by the edge extraction portion and in which the pixel of interest is present, based on a result of determination by the determination portion, and a raised shape detection portion that detects a raised shape based on a result of calculation by the shape feature value calculation portion.

A medical image processing apparatus of a second aspect according to the present invention is the medical image processing apparatus of the first aspect, wherein the determination portion performs a process of detecting whether each end portion of the edge present in the local region is tangent to any of the ends of the local region as processing for determining whether the local region is divided by at least part of the edge extracted by the edge extraction portion.

A medical image processing apparatus of a third aspect according to the present invention includes a three-dimensional model estimation portion that estimates a three-dimensional model of a living body tissue, based on a two-dimensional image of the living body tissue inputted from a medical image pickup apparatus, a local region setting portion that sets a local region centered on a voxel of interest in the three-dimensional model, a determination portion that determines whether the number of pieces of three-dimensional coordinate data included in the local region is larger than a predetermined threshold value, a shape feature value calculation portion that calculates a shape feature value at the voxel of interest using the pieces of three-dimensional coordinate data included in the local region if the number of pieces of three-dimensional coordinate data included in the local region is larger than the predetermined threshold value, based on a result of determination by the determination portion, and a raised shape detection portion that detects a raised shape based on a result of calculation by the shape feature value calculation portion.

A medical image processing apparatus of a fourth aspect according to the present invention is the medical image processing apparatus of the third aspect, further including an edge extraction portion that extracts an edge in the two-dimensional image, wherein the local region setting portion determines, based on a result of edge extraction by the edge extraction portion, whether the voxel of interest is a voxel corresponding to the edge in the two-dimensional image and changes a size of the local region depending on a result of the determination.

A medical image processing apparatus of a fifth aspect according to the present invention is the medical image processing apparatus of the fourth aspect, wherein the local region setting portion sets the size of the local region to a first size if the voxel of interest is not a voxel corresponding to the edge in the two-dimensional image and sets the size of the local region to a second size smaller than the first size if the voxel of interest is a voxel corresponding to the edge in the two-dimensional image.

A medical image processing apparatus of a sixth aspect not forming part of the present invention includes an edge extraction portion that extracts an edge in a two-dimensional image of a living body tissue inputted from a medical image pickup apparatus, based on the two-dimensional image of the living body tissue, a three-dimensional model estimation portion that estimates a three-dimensional model of the living body tissue, based on the two-dimensional image, a shape feature value calculation portion that calculates, as a shape feature value, a curvature of one edge of the two-dimensional image in the three-dimensional model, based on three-dimensional coordinate data of a portion corresponding to the one edge of the two-dimensional image, and a raised shape detection portion that detects a raised shape based on a result of calculation by the shape feature value calculation portion.

A medical image processing method of a first aspect according to the present invention includes an edge extraction step of extracting an edge in a two-dimensional image of a living body tissue inputted from a medical image pickup apparatus, based on the two-dimensional image, a three-dimensional model estimation step of estimating a three-dimensional model of the living body tissue, based on the two-dimensional image, a local region setting step of setting a local region centered on a pixel of interest in the two-dimensional image, a determination step of determining whether the local region is divided by at least part of the edge extracted in the edge extraction step, a shape feature value calculation step of calculating a shape feature value of the pixel of interest using three-dimensional coordinate data corresponding to, of the local region, a region which is not divided by the edge extracted in the edge extraction step and in which the pixel of interest is present, based on a result of determination in the determination step, and a raised shape detection step of detecting a raised shape based on a result of calculation in the shape feature value calculation step.

A medical image processing method of a second aspect according to the present invention is the medical image processing method of the first aspect, wherein the determination step comprises performing a process of detecting whether each end portion of the edge present in the local region is tangent to any of the ends of the local region as processing for determining whether the local region is divided by at least part of the edge extracted in the edge extraction step.

A medical image processing method of a third aspect according to the present invention includes a three-dimensional model estimation step of estimating a three-dimensional model of a living body tissue, based on a two-dimensional image of the living body tissue inputted from a medical image pickup apparatus, a local region setting step of setting a local region centered on a voxel of interest in the three-dimensional model, a determination step of determining whether the number of pieces of three-dimensional coordinate data included in the local region is larger than a predetermined threshold value, a shape feature value calculation step of calculating a shape feature value at the voxel of interest using the pieces of three-dimensional coordinate data included in the local region if the number of pieces of three-dimensional coordinate data included in the local region is larger than the predetermined threshold value, based on a result of determination in the determination step, and a raised shape detection step of detecting a raised shape based on a result of calculation in the shape feature value calculation step.

A medical image processing method of a fourth aspect according to the present invention is the medical image processing apparatus of the third aspect, further including an edge extraction step of extracting an edge in the two-dimensional image, wherein the local region setting step comprises determining, based on a result of edge extraction in the edge extraction step, whether the voxel of interest is a voxel corresponding to the edge in the two-dimensional image and changing a size of the local region depending on a result of the determination.

A medical image processing method of a fifth aspect according to the present invention is the medical image processing method of the fourth aspect, wherein the local region setting step includes setting the size of the local region to a first size if the voxel of interest is not a voxel corresponding to the edge in the two-dimensional image and setting the size of the local region to a second size smaller than the first size if the voxel of interest is a voxel corresponding to the edge in the two-dimensional image.

A medical image processing method of a sixth aspect not forming part of the present invention includes an edge extraction step of extracting an edge in a two-dimensional image of a living body tissue inputted from a medical image pickup apparatus, based on the two-dimensional image, a three-dimensional model estimation step of estimating a three-dimensional model of the living body tissue, based on the two-dimensional image, a shape feature value calculation step of calculating, as a shape feature value, a curvature of one edge of the two-dimensional image in the three-dimensional model, based on three-dimensional coordinate data of a portion corresponding to the one edge of the two-dimensional image, and a raised shape detection step of detecting a raised shape based on a result of calculation in the shape feature value calculation step.

### Brief Description of the Drawings

Fig. 1 is a diagram showing an example of an overall configuration of an endoscope system in which a medical image processing apparatus according to embodiments of the present invention is used;
Fig. 2 is a flow chart showing an example of a procedure for processing to be performed by the medical image processing apparatus in Fig. 1 in a first embodiment;
Fig. 3 is a view showing an example of an edge image acquired by the medical image processing apparatus in Fig. 1;
Fig. 4 is an enlarged view of one local region in the edge image in Fig. 3;
Fig. 5 is a schematic view showing a state when labeling is performed on the one local region in Fig. 4;
Fig. 6 is a view showing a correspondence between each region on which labeling is performed as in Fig. 5 and a portion in a three-dimensional model where three-dimensional coordinate data for the region is present;
Fig. 7 is a flow chart showing an example of a procedure for processing to be performed by the medical image processing apparatus in Fig. 1 in a second embodiment;
Fig. 8 is a flow chart showing an example different from the example in Fig. 7 of the procedure for processing to be performed by the medical image processing apparatus in Fig. 1 in the second embodiment; and
Fig. 9 is a flow chart showing an example of a procedure for processing to be performed by the medical image processing apparatus in Fig. 1 in a third embodiment.

### Best Mode for Carrying Out the Invention

### (First Embodiment)

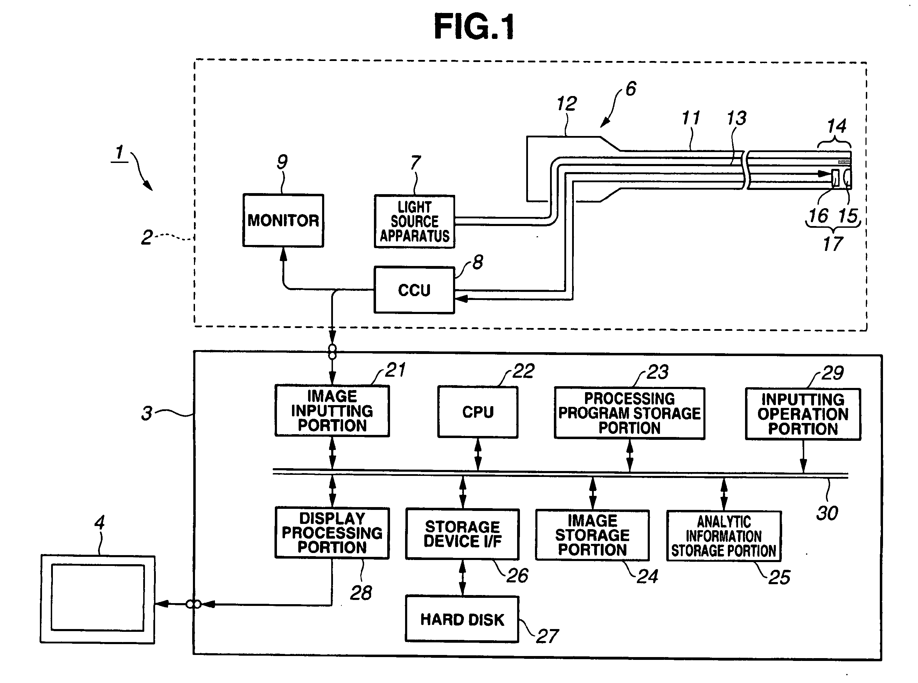

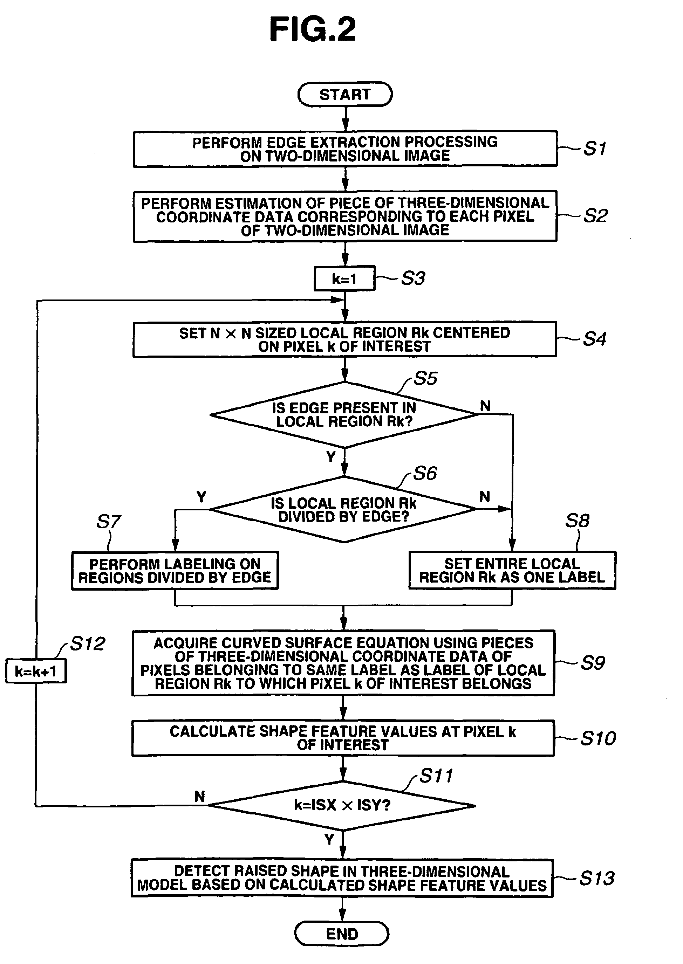

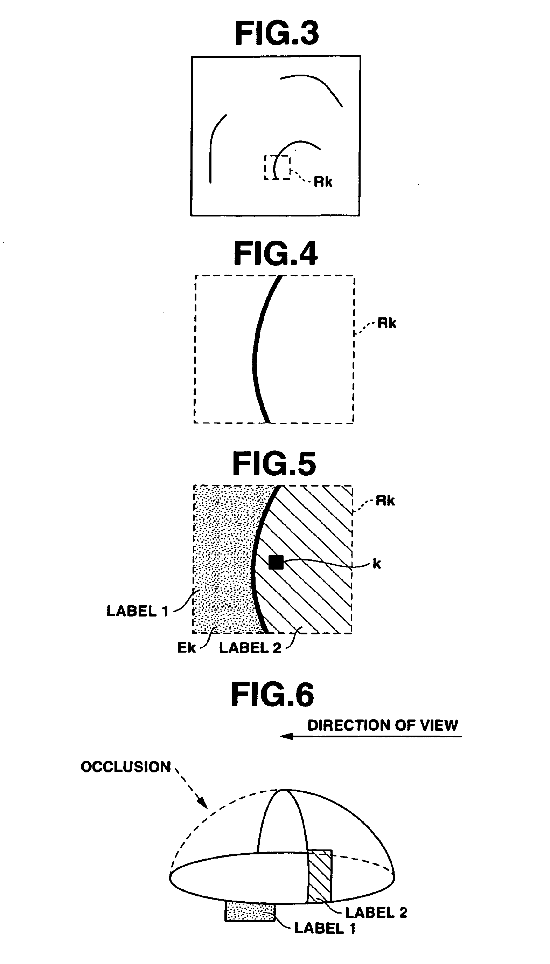

Figs. 1 to 6 relate to a first embodiment of the present invention. Fig. 1 is a diagram showing an example of an overall configuration of an endoscope system in which a medical image processing apparatus according to embodiments of the present invention is used. Fig. 2 is a flow chart showing an example of a procedure for processing to be performed by the medical image processing apparatus in Fig. 1 in a first embodiment. Fig. 3 is a view showing an example of an edge image acquired by the medical image processing apparatus in Fig. 1. Fig. 4 is an enlarged view of one local region in the edge image in Fig. 3. Fig. 5 is a schematic view showing a state when labeling is performed on the one local region in Fig. 4. Fig. 6 is a view showing a correspondence between each region on which labeling is performed as in Fig. 5 and a portion in a three-dimensional model where three-dimensional coordinate data for the region is present.

A main portion of an endoscope system 1 is configured to have a medical observation apparatus 2 which picks up an image of a subject and outputs a two-dimensional image of the subject, a medical image processing apparatus 3 which is composed of a personal computer or the like, performs image processing on a video signal of a two-dimensional image outputted from the medical observation apparatus 2, and outputs, as an image signal, the video signal having undergone the image processing, a monitor 4 which displays an image based on an image signal outputted from the medical image processing apparatus 3, as shown in Fig. 1.

A main portion of the medical observation apparatus 2 is configured to have an endoscope 6 which is inserted into a body cavity and picks up an image of a subject in the body cavity and outputs the image as an image pickup signal, a light source apparatus 7 which supplies illumination light for illuminating a subject, an image of which is picked up by the endoscope 6, a camera control unit (hereinafter abbreviated as CCU) 8 which performs various types of control on the endoscope 6 and performs signal processing on an image pickup signal outputted from the endoscope 6 and outputs the image pickup signal as a video signal of a two-dimensional image, and a monitor 9 which displays an image of a subject picked up by the endoscope 6 on the basis of a video signal of a two-dimensional image outputted from the CCU 8.

The endoscope 6 is configured to have an insertion portion 11 which is to be inserted into a body cavity and an operation portion 12 which is provided on a proximal end side of the insertion portion 11. A light guide 13 for transmitting illumination light supplied from the light source apparatus 7 is inserted through a portion from the proximal end side of the insertion portion 11 to a distal end portion 14 on a distal end side in the insertion portion 11.

A distal end side of the light guide 13 is arranged to the distal end portion 14 of the endoscope 6, and a rear end side is connected to the light source apparatus 7. Since the light guide 13 has the above-described configuration, illumination light supplied from the light source apparatus 7 is transmitted by the light guide 13 and is then emitted from an illumination window (not shown) provided at a distal end surface of the distal end portion 14 of the insertion portion 11. The emission of the illumination light from the illumination window (not shown) causes a living body tissue or the like as a subject to be illuminated.

An image pickup portion 17 having an objective optical system 15 which is attached to an observation window (not shown) adjacent to the illumination window (not shown) and an image pickup device 16 which is arranged at an image formation position of the objective optical system 15 and is composed of, e.g., a CCD (charge coupled device) is provided at the distal end portion 14 of the endoscope 6. With the configuration, after an image of a subject formed by the objective optical system 15 is picked up by the image pickup device 16, the image is outputted as an image pickup signal.

The image pickup device 16 is connected to the CCU 8 through a signal line. The image pickup device 16 is driven based on a driving signal outputted from the CCU 8 and outputs an image pickup signal to the CCU 8.

An image pickup signal inputted to the CCU 8 is subjected to signal processing in a signal processing circuit (not shown) provided in the CCU 8, is converted into a video signal of a two-dimensional image, and is outputted. The video signal of the two-dimensional image outputted from the CCU 8 is outputted to the monitor 9 and the medical image processing apparatus 3. With the operation, an image of a subject based on video signals outputted from the CCU 8 is displayed as a two-dimensional image on the monitor 9.

The medical image processing apparatus 3 has an image inputting portion 21 which performs A/D conversion on a video signal of a two-dimensional image outputted from the medical observation apparatus 2 and outputs the video signal, a CPU 22 serving as a central processing unit which performs image processing on a video signal outputted from the image inputting portion 21, a processing program storage portion 23 to which a processing program relating to the image processing has been written, an image storage portion 24 which stores a video signal outputted from the image inputting portion 21 and the like, and an information storage portion 25 which stores image data as a result of image processing by the CPU 22 and the like.

The medical image processing apparatus 3 also has a storage device interface 26, a hard disk 27 as a storage device which stores image data as a result of image processing by the CPU 22 and the like through the storage device interface 26, a display processing portion 28 which performs, on the basis of image data as a result of image processing by the CPU 22, display processing for displaying the image data as an image on the monitor 4 and outputs the image data having undergone the display processing as an image signal, and an inputting operation portion 29 through which a user can input a parameter in image processing performed by the CPU 22 and an operation instruction to the medical image processing apparatus 3 and which is composed of a keyboard and the like. The monitor 4 displays an image based on image signals outputted from the display processing portion 28.

Note that the image inputting portion 21, CPU 22, processing program storage portion 23, image storage portion 24, information storage portion 25, storage device interface 26, display processing portion 28, and inputting operation portion 29 of the medical image processing apparatus 3 are connected to each other through a data bus 30.

Operation of the endoscope system 1 will be described.

First, a user inserts the insertion portion 11 of the endoscope 6 into a body cavity. When the insertion portion 11 is inserted into the body cavity by the user, an image of a living body tissue as a subject is picked up by the image pickup portion 17 provided at the distal end portion 14. The image of the living body tissue picked up by the image pickup portion 17 is outputted as image pickup signals to the CCU 8.

The CCU 8 performs signal processing on the image pickup signals outputted from the image pickup device 16 of the image pickup portion 17 in the signal processing circuit (not shown), thereby converting the image pickup signals into video signals of a two-dimensional image and outputting the video signals. The monitor 9 displays the image of the living body tissue as the two-dimensional image on the basis of the video signals outputted from the CCU 8. The CCU 8 also outputs the video signals of the two-dimensional image acquired by performing signal processing on the image pickup signals outputted from the image pickup device 16 of the image pickup portion 17 to the medical image processing apparatus 3.

The video signals of the two-dimensional image outputted to the medical image processing apparatus 3 are A/D-converted in the image inputting portion 21 and are then inputted to the CPU 22.

The CPU 22 performs edge extraction processing based on a gray level ratio between adjacent pixels on the two-dimensional image outputted from the image inputting portion 21 (step S1 of Fig. 2). With the processing, the CPU 22 acquires, e.g., an image as shown in Fig. 3 as an edge image corresponding to the two-dimensional image.

Note that the edge extraction processing is not limited to one based on a gray level ratio between adjacent pixels. For example, one to be performed using a band-pass filter corresponding to red components of the two-dimensional image may be adopted.

The CPU 22 performs processing such as geometrical transformation based on luminance information and the like of the two-dimensional image using, e.g., ShapeFromShading on the basis of the two-dimensional image outputted from the image inputting portion 21, thereby estimating a piece of three-dimensional coordinate data corresponding to each pixel of the two-dimensional image (step S2 of Fig. 2).

Assume in the present embodiment that a two-dimensional image outputted from the image inputting portion 21 is an image which has ISX pixels in a horizontal direction and ISY pixels in a vertical direction, i.e., an ISX × ISY sized image.

The CPU 22 sets, of pixels of the edge image, a pixel k of interest to 1 (step S3 of Fig. 2) and then sets an N × N (e.g., 15 × 15) sized local region Rk centered on the pixel k of interest (step S4 of Fig. 2). Assume that the pixel k of interest is a variable defined by 1 ≤ k ≤ ISX × ISY. Assume also that a value of N is not more than each of the number ISX of pixels in the horizontal direction in the two-dimensional image and the number ISY of pixels in the vertical direction in the two-dimensional image.

After the CPU 22 sets the local region Rk in, e.g., a manner as shown in Figs. 3 and 4 by the process in step S4 of Fig. 2, the CPU 22 determines whether an edge is present in the local region Rk (step S5 of Fig. 2). If the CPU 22 detects that an edge is present in the local region Rk, the CPU 22 further determines whether the local region Rk is divided by the edge (step S6 of Fig. 2).

If the CPU 22 detects in the process in step S5 of Fig. 2 that no edge is present in the local region Rk, the CPU 22 performs a process in step S8 of Fig. 2 (to be described later). If the CPU 22 detects in the process in step S6 of Fig. 2 that the local region Rk is not divided by the edge in the local region Rk, the CPU 22 performs the process in step S8 of Fig. 2 (to be described later).

The CPU 22 determines in the process in step S6 of Fig. 2 whether the local region Rk is divided, by detecting whether each end of the edge present in the local region Rk is tangent to any end portion of the local region Rk.

More specifically, the CPU 22 detects that two ends of an edge Ek are each tangent to an end portion of the local region Rk in, for example, the local region Rk shown in Fig. 5 and determines on the basis of a result of the detection that the local region Rk is divided into two regions.

If the CPU 22 detects in the process in step S6 of Fig. 2 that the local region Rk is divided by the edge present in the local region Rk, labeling is performed on each of the regions (in the local region Rk) divided by the edge (step S7 of Fig. 2).

More specifically, the CPU 22 sets the region on a left side of the edge Ek as label 1 and sets the region on a right side of the edge Ek as label 2.

The regions divided by the edge Ek in the local region Rk are estimated as pieces of three-dimensional coordinate data present on discontiguous planes on opposite sides of the edge Ek when a three-dimensional model is estimated from the two-dimensional image. The CPU 22 detects on the basis of the pieces of three-dimensional coordinate data present on the discontiguous planes acquired by the labeling shown as the process in step S7 of Fig. 2 that occlusion has occurred in the three-dimensional model. The CPU 22, for example, regards a portion set as label 1 in Fig. 5 as a piece of three-dimensional coordinate data in which a portion above an edge corresponds to an occlusion-related portion, as shown in Fig. 6, and performs three-dimensional model estimation. Also, the CPU 22, for example, regards a portion set as label 2 in Fig. 5 as a piece of three-dimensional coordinate data in which a portion on a left side of an edge corresponds to the occlusion-related portion, as shown in Fig. 6, and performs three-dimensional model estimation.

The CPU 22 sets the entire local region Rk as one label either if the CPU 22 detects in the process in step S5 of Fig. 2 that no edge is present in the local region Rk or if the CPU 22 detects in the process in step S6 of Fig. 2 that the local region Rk is not divided by the edge in the local region Rk (step S8 of Fig. 2).

After that, the CPU 22 acquires a curved surface equation using pieces of three-dimensional coordinate data of pixels in a region with a same label as a label to which the pixel k of interest belongs of the local region Rk (step S9 of Fig. 2).

More specifically, the CPU 22 acquires a curved surface equation using pieces of three-dimensional coordinate data of pixels in the region with label 2 to which the pixel k of interest belongs in, for example, the local region Rk shown in Fig. 5. If the entire local region Rk is set as one label in the process in step S8 of Fig. 2, the CPU 22 acquires a curved surface equation using pieces of three-dimensional coordinate data of pixels present in the entire local region Rk.

The CPU 22 calculates shape feature values at the pixel k of interest on the basis of the curved surface equation acquired in the process in step S9 of Fig. 2 (step S10 of Fig. 2). Note that, in the present embodiment, the CPU 22 calculates a ShapeIndex value and a Curvedness value as the shape feature values. A ShapeIndex value and a Curvedness value described above can be calculated from a curved surface equation using a same method as a method described in, e.g., US Patent Application Publication No. 20030223627. For the reason, a description of a method for calculating a ShapeIndex value and a Curvedness value will be omitted in the description of the present embodiment.

The CPU 22 can accurately calculate the shape feature values at the pixel k of interest using pieces of three-dimensional coordinate data with relatively high estimation result reliability without use of pieces of data corresponding to the occlusion in the three-dimensional model, i.e., pieces of three-dimensional coordinate data with relatively low estimation result reliability, by performing the processes in steps S9 and S10 of Fig. 2.

After the CPU 22 calculates the shape feature values of the pixel k of interest in the process in step S10 of Fig. 2, the CPU 22 determines whether a value of the pixel k of interest is ISX × ISY (step S 11 of Fig. 2). If the value of the pixel k of interest is ISX × ISY, the CPU 22 proceeds to perform a process in step S13 of Fig. 2 (to be described later). On the other hand, if the value of the pixel k of interest is not ISX × ISY, the CPU 22 sets the value of the pixel k of interest to k + 1 (step S12 of Fig. 2) and then performs the series of processes from steps S4 to S 11 of Fig. 2 again.

After the CPU 22 completes the calculation of shape feature values of pixels in the edge image in the process in step S 11 of Fig. 2, the CPU 22 performs threshold processing based on the calculated shape feature values. With the processing, the CPU 22 detects a raised shape in the three-dimensional model (step S13 of Fig. 2).

More specifically, for example, if a threshold value Sth for a Shapelndex value is set to 0.9, and a threshold value Cth for a Curvedness value is set to 0.2, the CPU 22 detects, as a (local) raised shape (caused by a lesion such as a polyp), pieces of three-dimensional coordinate data having shape feature values larger than the threshold value Sth and larger than the threshold value Cth.

Note that a result of detecting a raised shape may be indicated to a user, for example, by coloration of a corresponding portion of a two-dimensional image (or three-dimensional model) displayed on the monitor 4 or by a symbol and (or) characters pointing to the corresponding portion of the two-dimensional image (or three-dimensional model) displayed on the monitor 4.

As has been described above, the medical image processing apparatus 3 of the present embodiment performs calculation of shape feature values using only pieces of three-dimensional coordinate data with relatively high estimation result reliability in the series of processes described as the processes shown in Fig. 2. Consequently, the medical image processing apparatus 3 of the present embodiment is capable of making an efficiency of detecting a raised shape higher than ever before when a three-dimensional model is estimated on the basis of a two-dimensional image acquired by the endoscope.

### (Second Embodiment)

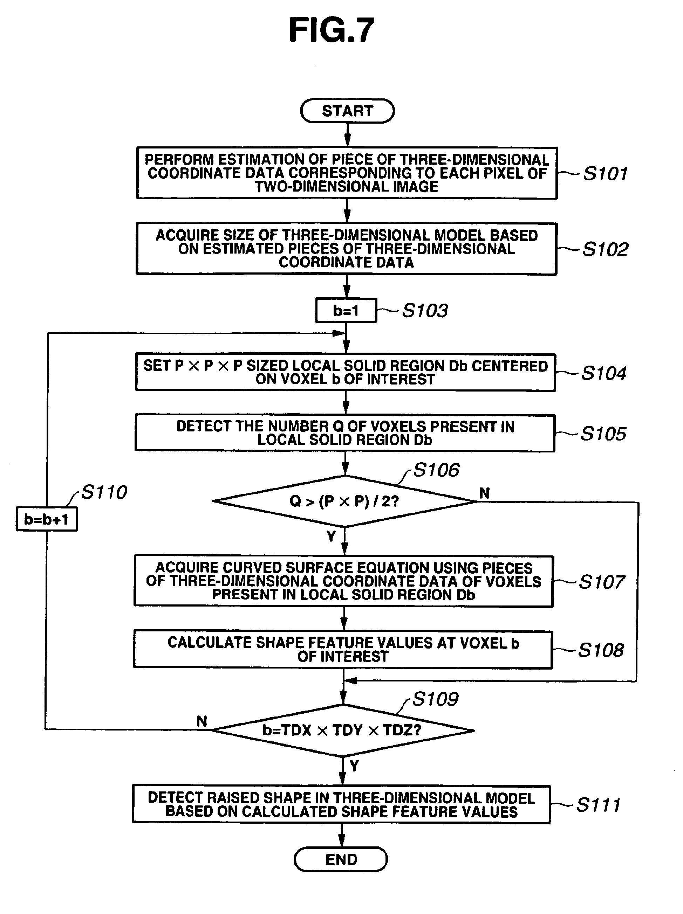

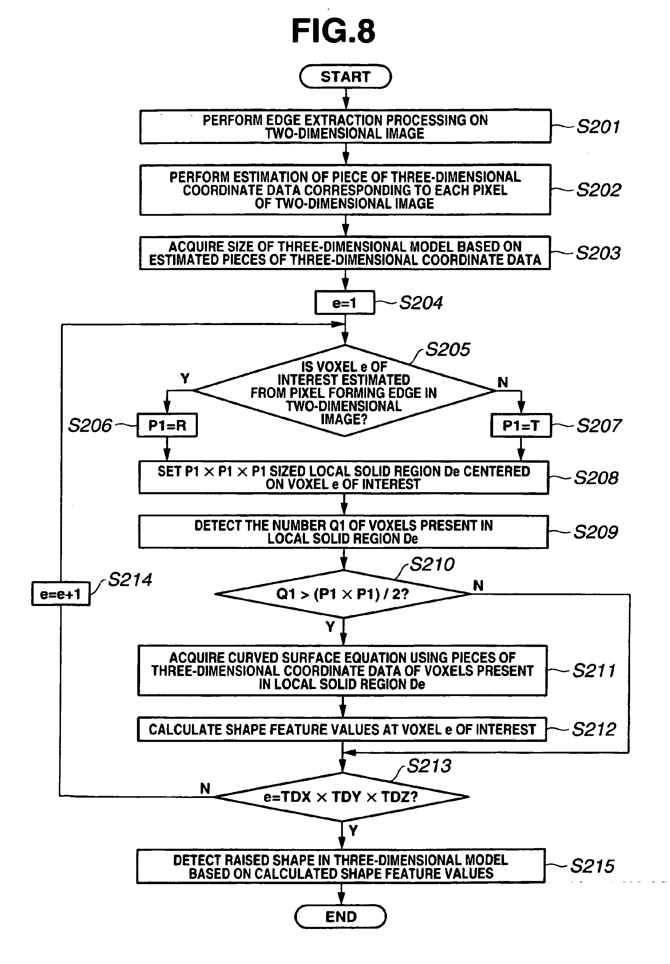

Figs. 7 and 8 relate to a second embodiment of the present invention. Fig. 7 is a flow chart showing an example of a procedure for processing to be performed by the medical image processing apparatus in Fig. 1 in a second embodiment. Fig. 8 is a flow chart showing an example different from the example in Fig. 7 of the procedure for the processing to be performed by the medical image processing apparatus in Fig. 1 in the second embodiment.

Note that a detailed description of a portion with a same configuration as that of the first embodiment will be omitted. A configuration of an endoscope system 1 to be used in the present embodiment is the same as that of the first embodiment.

Image processing operation to be performed in a medical image processing apparatus 3 will be described.

A CPU 22 performs processing such as geometrical transformation based on luminance information and the like of a two-dimensional image outputted from an image inputting portion 21 using, e.g., ShapeFromShading on the basis of the two-dimensional image. With the processing, the CPU 22 performs estimation of a piece of three-dimensional coordinate data corresponding to each pixel of the two-dimensional image (step S101 of Fig. 7).

After that, the CPU 22 calculates a difference between a maximum value and a minimum value of an x coordinate, a difference between a maximum value and a minimum value of a y coordinate, and a difference between a maximum value and a minimum value of a z coordinate on the basis of the pieces of three-dimensional coordinate data estimated in the process in step S101 of Fig. 7, thereby acquiring a size of a three-dimensional model (step S102 of Fig. 7).

Assume that the CPU 22 acquires TDX × TDY × TDZ as the size of the three-dimensional model by detecting that TDX voxels are present in an x-axis direction (horizontal direction), that TDY voxels are present in a y-axis direction (depth direction), and that TDZ voxels are present in a z-axis direction (height direction), in the process in step S102 of Fig. 7.

The CPU 22 sets, of voxels in the three-dimensional model, a voxel b of interest to 1 (step S103 of Fig. 7) and then sets a P × P × P (e.g., 15 × 15 × 15) sized local solid region Db centered on the voxel b of interest (step S104 of Fig. 7). Assume that the voxel b of interest is a variable defined by 1 ≤ b ≤ TDX × TDY × TDZ. Assume also that a value of P is not more than each of the number TDX of voxels in the x-axis direction in the three-dimensional model, the number of TDY of voxels in the y-axis direction in the three-dimensional model, and the number TDZ of voxels in the z-axis direction in the three-dimensional model.

The CPU 22 detects the number Q of voxels present in the local solid region Db (step S105 of Fig. 7) and then compares a value of the number Q of voxels with a value calculated by performing the operation (P × P)/2 (step S 106 of Fig. 7).

If the CPU 22 detects that the value of the number Q of voxels is larger than the value calculated by performing the operation (P × P)/2, the CPU 22 further performs a process of acquiring a curved surface equation using pieces of three-dimensional coordinate data of the voxels present in the local solid region Db (step S107 of Fig. 7). On the other hand, if the CPU 22 detects that the value of the number Q of voxels is not more than the value calculated by performing the operation (P × P)/2, the CPU 22 performs a process in step S109 of Fig. 7 (to be described later).

Note that the value of the number Q of voxels described above is a value indicating the number of voxels for which pieces of three-dimensional coordinate data (with relatively high reliability) are estimated by the CPU 22. The value of (P × P)/2 described above is a value indicating a lower limit of the number of voxels required for the CPU 22 to acquire a curved surface equation. That is, the CPU 22 determines by the process in step S106 of Fig. 7 described above whether the local solid region Db is a region for which a curved surface equation can be acquired.

The CPU 22 calculates shape feature values at the voxel b of interest on the basis of the curved surface equation acquired in the process in step S107 of Fig. 7 (step S108 of Fig. 7). Note that, in the present embodiment, the CPU 22 calculates a ShapeIndex value and a Curvedness value as the shape feature values. A ShapeIndex value and a Curvedness value described above can be calculated from a curved surface equation using a same method as the method described in, e.g., US Patent Application Publication No. 20030223627. For the reason, a description of a method for calculating a ShapeIndex value and a Curvedness value will be omitted in the description of the present embodiment.

The CPU 22 can accurately calculate the shape feature values at the voxel b of interest while excluding, from processing, pieces of data at a portion corresponding to occlusion in the three-dimensional model, i.e., a region having many pieces of three-dimensional coordinate data with relatively low estimation result reliability and including, in processing, a region having many pieces of three-dimensional coordinate data with relatively high estimation result reliability, by performing the processes in steps S106 to S 108 of Fig. 7.

After the CPU 22 calculates the shape feature values of the voxel b of interest in the process in step S108 of Fig. 7, the CPU 22 determines whether a value of the voxel b of interest is TDX × TDY × TDZ (step S109 of Fig. 7). If the value of the voxel b of interest is TDX × TDY × TDZ, the CPU 22 proceeds to perform a process in step S111 of Fig. 7 (to be described later). On the other hand, if the value of the voxel b of interest is not TDX × TDY × TDZ, the CPU 22 sets the value of the voxel b of interest to b + 1 (step S 110 of Fig. 7) and then performs the above-described series of processes from steps S104 to S109 of Fig. 7 again.

After the CPU 22 completes the calculation of shape feature values of voxels in the three-dimensional model in the process in step S110 of Fig. 7, the CPU 22 performs threshold processing based on the calculated shape feature values. With the processing, the CPU 22 detects a raised shape in the three-dimensional model (step S111 of Fig. 7).

More specifically, for example, if a threshold value Sth1 for a ShapeIndex value is set to 0.9, and a threshold value Cth1 for a Curvedness value is set to 0.2, the CPU 22 detects, as a (local) raised shape (caused by a lesion such as a polyp), pieces of three-dimensional coordinate data having shape feature values larger than the threshold value Sth1 and larger than the threshold value Cth1.

Note that a result of detecting a raised shape may be indicated to a user, for example, by coloration of a corresponding portion of a two-dimensional image (or three-dimensional model) displayed on a monitor 4 or by a symbol and (or) characters pointing to the corresponding portion of the two-dimensional image (or three-dimensional model) displayed on the monitor 4.

As has been described above, the medical image processing apparatus 3 of the present embodiment performs calculation of shape feature values only in a region having many pieces of three-dimensional coordinate data with relatively high estimation result reliability in the series of processes described as the processes shown in Fig. 7. Consequently, the medical image processing apparatus 3 of the present embodiment is capable of making an efficiency of detecting a raised shape higher than ever before when a three-dimensional model is estimated on the basis of a two-dimensional image acquired by an endoscope.

Note that, in the present embodiment, the CPU 22 is not limited to one which performs a process of detecting a region for which a curved surface equation is to be acquired using a local solid region of fixed size. For example, the CPU 22 may be one which performs a process of detecting a region for which a curved surface equation is to be acquired while changing a size of a local solid region depending on whether a voxel of interest is present on an edge.

In that case, the CPU 22 first performs same edge extraction processing as step S1 of Fig. 2, which has already been described in the explanation of the first embodiment, on a two-dimensional image (step S201 of Fig. 8). With the processing, the CPU 22 detects a portion where an edge is present in the two-dimensional image.

The CPU 22 performs estimation of pieces of three-dimensional coordinate data corresponding to pixels of the two-dimensional image by same processes as the processes in steps S101 and S102 of Fig. 7 and acquires a size of a three-dimensional model on the basis of the pieces of three-dimensional coordinate data (steps S202 and S203 of Fig. 8).

The CPU 22 sets, of voxels in the three-dimensional model, a voxel e of interest to 1 (step S204 of Fig. 8) and then determines on the basis of the piece of three-dimensional coordinate data of the voxel e of interest whether the voxel e of interest is estimated from a pixel constituting the edge in the two-dimensional image (step S205 of Fig. 8).

If the voxel e of interest is estimated from a pixel constituting the edge in the two-dimensional image, the CPU 22 sets a variable P1 to R (step S206 of Fig. 8) and then proceeds to perform a process in step S208 of Fig. 8 (to be described later). On the other hand, if the voxel e of interest is not estimated from a pixel constituting the edge in the two-dimensional image, the CPU 22 sets the variable P1 to T (step S207 of Fig. 8) and then proceeds to perform the process in step S208 (to be described later).

Note that the value R to be set in the process in step S206 of Fig. 8 is set to a value (e.g., R = T/2) smaller than the value T to be set in the step S207 of Fig. 8. That is, the CPU 22 can acquire more regions for which curved surface equations are to be acquired than a case where the series of processes shown in Fig. 7 is performed, by changing a size of a local solid region depending on whether the voxel of interest is present on an edge in the processes in steps S205 to S207 of Fig. 8.

The CPU 22 sets a P1 × P1 × P1 sized local solid region De centered on the voxel e of interest and detects the number Q1 of voxels present in the local solid region De by same processes as the processes in steps S104 and S105 of Fig. 7 (steps S208 and S209 of Fig. 8).

After that, the CPU 22 compares a value of the number Q1 of voxels with a value calculated by performing the operation (P1 × P1)/2 (step S210 of Fig. 8).

If the CPU 22 detects that the value of the number Q1 of voxels is larger than the value calculated by performing the operation (P1 × P1)/2, the CPU 22 further performs a process of acquiring a curved surface equation using pieces of three-dimensional coordinate data of the voxels present in the local solid region De (step S211 of Fig. 8). On the other hand, if the CPU 22 detects that the value of the number Q1 of voxels is not more than the value calculated by performing the operation (P1 × P)/2, the CPU 22 performs a process in step S213 of Fig. 8 (to be described later).

Note that the value of the number Q1 of voxels described above is a value indicating the number of voxels for which pieces of three-dimensional coordinate data (with relatively high reliability) are estimated by the CPU 22. The value of (P1 × P1)/2 described above is a value indicating a lower limit of the number of voxels required for the CPU 22 to acquire a curved surface equation. That is, the CPU 22 determines by the process in step S210 of Fig. 8 described above whether the local solid region De is a region for which a curved surface equation can be acquired.

The CPU 22 calculates shape feature values at the voxel e of interest (e.g., a Shapelndex value and a Curvedness value as the shape feature values) on the basis of the curved surface equation acquired in the process in step S211 of Fig. 8 (step S212 of Fig. 8).

After that, the CPU 22 determines whether a value of the voxel e of interest is TDX × TDY × TDZ (step S213 of Fig. 8). If the value of the voxel e of interest is TDX × TDY × TDZ, the CPU 22 proceeds to perform a process in step S215 of Fig. 8 (to be described later). On the other hand, if the value of the voxel e of interest is not TDX × TDY × TDZ, the CPU 22 sets the value of the voxel e of interest to e + 1 (step S214 of Fig. 8) and then performs the above-described series of processes from steps S205 to S213 in Fig. 8 again.

After the CPU 22 completes the calculation of shape feature values of voxels in the three-dimensional model in the process in step S213 of Fig. 8, the CPU 22 further performs threshold processing based on the calculated shape feature values. With the processing, the CPU 22 detects a raised shape in the three-dimensional model (step S215 of Fig. 8).

More specifically, for example, if a threshold value Sth2 for a ShapeIndex value is set to 0.9, and a threshold value Cth2 for a Curvedness value is set to 0.2, the CPU 22 detects, as a (local) raised shape (caused by a lesion such as a polyp), pieces of three-dimensional coordinate data having shape feature values larger than the threshold value Sth2 and larger than the threshold value Cth2.

Note that a result of detecting a raised shape may be indicated to a user, for example, by coloration of a corresponding portion of a two-dimensional image (or three-dimensional model) displayed on the monitor 4 or by a symbol and (or) characters pointing to the corresponding portion of the two-dimensional image (or three-dimensional model) displayed on the monitor 4.

As has been described above, the medical image processing apparatus 3 of the present embodiment is capable of making an efficiency of detecting a raised shape higher than a case where the series of processes shown in Fig. 7 is performed, by performing the series of processes shown in Fig. 8.

### (Third Embodiment)

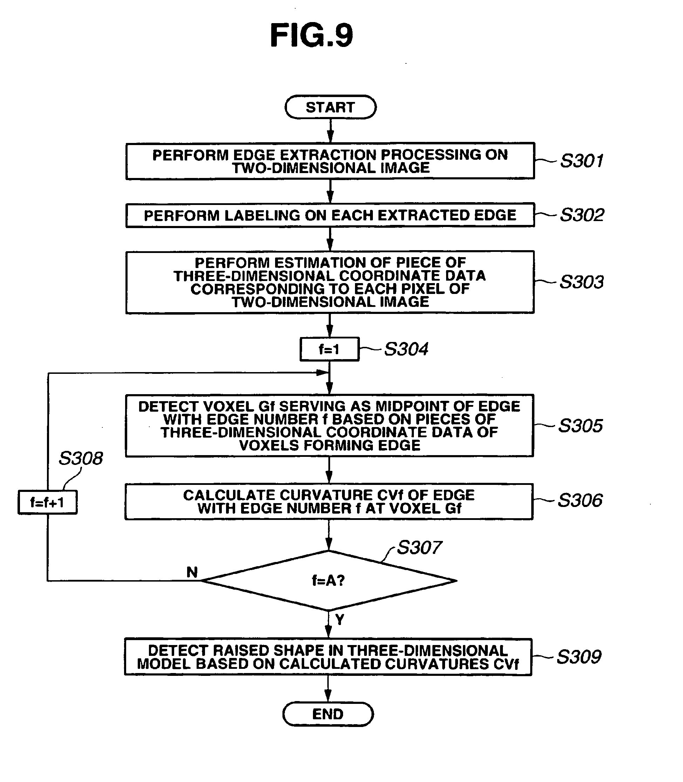

Fig. 9 relates to a third embodiment of the present invention. Fig. 9 is a flow chart showing an example of a procedure for processing to be performed by the medical image processing apparatus in Fig. 1 in the third embodiment.

Note that a detailed description of a portion with a same configuration as that of the first and second embodiments will be omitted. A configuration of an endoscope system 1 to be used in the present embodiment is the same as that of the first and second embodiments.

Image processing operation to be performed in a medical image processing apparatus 3 will be described.

A CPU 22 first performs same edge extraction processing as step S1 of Fig. 2, which has already been described in the explanation of the first embodiment, on a two-dimensional image and performs labeling on each extracted edge (steps S301 and S302 of Fig. 9).

Note that, in the present embodiment, the CPU 22 extracts A edges from a two-dimensional image and assigns labels numbered 1 through A to the extracted edges, by performing processes in steps S301 and S302 of Fig. 9.

After that, the CPU 22 performs estimation of pieces of three-dimensional coordinate data corresponding to pixels of the two-dimensional image by a same process as the process in step S101 of Fig. 7 (step S303 of Fig. 9).

The CPU 22 sets an edge number f to 1 (step S304 of Fig. 9) and then detects a voxel Gf serving as a midpoint of an edge with the edge number f on the basis of pieces of three-dimensional coordinate data of voxels constituting the edge (step S305 of Fig. 9).

The CPU 22 calculates, as a shape feature value, a curvature CVf of the edge with the edge number f at the voxel Gf detected by the process in step S305 of Fig. 9 (step S306 of Fig. 9).

The CPU 22 determines whether a value of the edge number f is A (step S307 of Fig. 9). If the value of the edge number f is A, the CPU 22 proceeds to perform a process in step S309 of Fig. 9 (to be described later). On the other hand, if the value of the edge number f is not A, the CPU 22 sets the value of the edge number f to f + 1 (step S308 of Fig. 9) and then performs the series of processes from steps S305 to S307 of Fig. 9 again.

After the CPU 22 completes the calculation of the curvature CVf of each edge in the three-dimensional model by the processes in steps S305 to S308 of Fig. 9, the CPU 22 further performs threshold processing based on the calculated curvature CVf. With the processing, the CPU 22 detects a raised shape in the three-dimensional model (step S309 of Fig. 9).

More specifically, for example, if a threshold value CVth for a curvature is set to 0.2, the CPU 22 detects, as an edge caused by a raised shape, pieces of three-dimensional coordinate data including a curvature larger than the threshold value CVth.

Note that a result of detecting a raised shape may be indicated to a user, for example, by coloration of a corresponding portion of a two-dimensional image (or three-dimensional model) displayed on a monitor 4 or by a symbol and (or) characters pointing to the corresponding portion of the two-dimensional image (or three-dimensional model) displayed on the monitor 4.

As has been described above, the medical image processing apparatus 3 of the present embodiment performs detection of a raised shape on the basis of shape feature values indicating a shape of an edge in the series of processes described as the processes shown in Fig. 9.

Consequently, the medical image processing apparatus 3 of the present embodiment is capable of making an efficiency of detecting a raised shape faster and higher than ever before when a three-dimensional model is estimated on the basis of a two-dimensional image acquired by an endoscope.

Note that the present invention is not limited to the embodiments described above. Various changes and applications are of course possible without departing from scope of the invention.

## Claims

1. A medical image processing apparatus (3) for estimating a three dimensional model of a living tissue, comprising:
an edge extraction portion that extracts an edge image based on a two-dimensional input image of a living body tissue, inputted from a medical image pickup apparatus (2);
a three-dimensional coordinate data estimation portion that estimates three-dimensional coordinate data for each pixel of the two-dimensional input image of the living body tissue;
a local region setting portion that sets a local region centered on a pixel of interest in the two-dimensional edge image;
a determination portion that determines whether the local region is divided into two regions by at least part of the edge extracted by the edge extraction portion;
a labeling portion for labeling each of said two regions in the local region divided by the edge or setting the entire local region as one label if the local region is not divided by the edge in the local region;
a shape feature value calculation portion that calculates a shape feature value of the pixel of interest using three-dimensional coordinate data of the local region corresponding to a region with the same label as the label to which the pixel of interest belongs in the local region; and
a raised shape detection portion that detects a raised shape based on a result of calculation by the shape feature value calculation portion.

2. The medical image processing apparatus (3) according to claim 1, wherein the determination portion performs a process of detecting whether each end of the edge present in the local region is tangent to any of the ends of the local region as processing for determining whether the local region is divided by at least part of the edge extracted by the edge extraction portion.

3. A medical image processing apparatus (3) for estimating a three dimensional model of a living tissue, comprising:
a three-dimensional coordinate data estimation portion that estimates three-dimensional coordinate data for each pixel of a two-dimensional input image of a living body tissue, inputted from a medical image pickup apparatus (2);
a three-dimensional model size determination portion for determining a size of a three-dimensional model in voxels, based on the three-dimensional coordinate data;
a local region setting portion that sets a local region centered on a voxel of interest in the three-dimensional coordinate data;
a determination portion that determines whether the number of pieces of three-dimensional coordinate data included in the local region is larger than a predetermined threshold value;
a shape feature value calculation portion that calculates a shape feature value at the voxel of interest using the pieces of three-dimensional coordinate data included in the local region if the number of pieces of three-dimensional coordinate data included in the local region is larger than the predetermined threshold value, based on a result of determination by the determination portion; and
a raised shape detection portion that detects a raised shape, based on a result of calculation by the shape feature value calculation portion.

4. The medical image processing apparatus (3) according to claim 3, further comprising:
an edge extraction portion that extracts an edge in the two-dimensional image, wherein
the local region setting portion determines, based on a result of edge extraction by the edge extraction portion, whether the voxel of interest is a voxel corresponding to the edge in the two-dimensional image and changes a size of the local region depending on a result of the determination.

5. The medical image processing apparatus (3) according to claim 4, wherein the local region setting portion sets the size of the local region to a first size if the voxel of interest is not a voxel corresponding to the edge in the two-dimensional image and sets the size of the local region to a second size smaller than the first size if the voxel of interest is a voxel corresponding to the edge in the two-dimensional image.

6. A medical image processing method for estimating a three dimensional model of a living tissue, comprising:
an edge extraction step of extracting an edge image based on a two-dimensional input image of a living body tissue, inputted from a medical image pickup apparatus (2);
a three-dimensional coordinate data estimation step of estimating a three-dimensional coordinate data for each pixel of the two-dimensional input image of the living body tissue;
a local region setting step of setting a local region centered on a pixel of interest in the two-dimensional edge image;
a determination step of determining whether the local region is divided into two regions by at least part of the edge extracted in the edge extraction step;
a labeling step of labeling each of said two regions in the local region divided by the edge or setting the entire local region as one label if the local region is not divided by the edge in the local region;
a shape feature value calculation step of calculating a shape feature value of the pixel of interest using three-dimensional coordinate data of the local region corresponding to a region with the same label as the label to which the pixel of interest belongs in the local region; and
a raised shape detection step of detecting a raised shape based on a result of calculation in the shape feature value calculation step.

7. The medical image processing method according to claim 6, wherein the determination step comprises performing a process of detecting whether each end of the edge present in the local region is tangent to any of the ends of the local region as processing for determining whether the local region is divided by at least part of the edge extracted in the edge extraction step.

8. A medical image processing method for estimating a three dimensional model of a living tissue, comprising:
a three-dimensional coordinate data estimation step of estimating three-dimensional coordinate data for each pixel of a two-dimensionalinput image of a living body tissue, inputted from a medical image pickup apparatus (2);
a three-dimensional model size determination step of determining a size of a three-dimensional model in voxels, based on the three-dimensional coordinate data;
a local region setting step of setting a local region centered on a voxel of interest in the three-dimensional coordinate data;
a determination step of determining whether the number of pieces of three-dimensional coordinate data included in the local region is larger than a predetermined threshold value;
a shape feature value calculation step of calculating a shape feature value at the voxel of interest using the pieces of three-dimensional coordinate data included in the local region if the number of pieces of three-dimensional coordinate data included in the local region is larger than the predetermined threshold value, based on a result of determination in the determination step; and
a raised shape detection step of detecting a raised shape, based on a result of calculation in the shape feature value calculation step.

9. The medical image processing method according to claim 8, further comprising:
an edge extraction step of extracting an edge in the two-dimensional image, wherein
the local region setting step comprises determining, based on a result of edge extraction in the edge extraction step, whether the voxel of interest is a voxel corresponding to the edge in the two-dimensional image and changing a size of the local region depending on a result of the determination.

10. The medical image processing method according to claim 9, wherein the local region setting step comprises setting the size of the local region to a first size if the voxel of interest is not a voxel corresponding to the edge in the two-dimensional image and setting the size of the local region to a second size smaller than the first size if the voxel of interest is a voxel corresponding to the edge in the two-dimensional image.

## Patentansprüche

1. Medizinische Bildverarbeitungsvorrichtung (3) zum Abschätzen eines dreidimensionalen Modells von einem lebenden Gewebe mit:
einem Randextraktionsteil, das auf der Grundlage eines zweidimensionalen Eingangsbildes von einem lebenden Körpergewebe ein Randbild extrahiert, das von einer medizinischen Bildaufnahmevorrichtung (2) eingegeben wird;
einem Dreidimensional-Koordinatendaten-Abschätzteil, das für jedes Pixel des zweidimensionalen Eingangsbildes vom lebenden Körpergewebe dreidimensionale Koordinatendaten abschätzt;
einem Lokalbereich-Einstellteil, das einen Lokalbereich auf ein interessierendes Pixel in dem zweidimensionalen Randbild mittig einstellt;
einem Bestimmungsteil, das bestimmt, ob der Lokalbereich durch mindestens einen Teil des durch das Randextraktionsteil extrahierten Randes in zwei Bereiche geteilt wird;
einem Kennzeichnungsteil zum Kennzeichnen eines jeden der beiden Bereiche in dem Lokalbereich, der durch den Rand geteilt wird, oder zum Einstellen des gesamten Lokalbereichs als eine Kennzeichnung, wenn der Lokalbereich nicht durch den Rand in dem Lokalbereich geteilt wird;
einem Formmerkmalswert-Berechnungsteil, das einen Formmerkmalswert des interessierenden Pixels unter Verwendung dreidimensionaler Koordinatendaten des Lokalbereichs berechnet, die zu einem Bereich mit derselben Kennzeichnung gehören wie die Kennzeichnung, zu der das interessierende Pixel in dem dem Lokalbereich gehört; und
einem Formerhöhung-Ermittlungsteil, das eine erhöhte Form auf der Grundlage eines Ergebnisses der Berechnung durch das Formmerkmalswert-Berechnungsteil ermittelt.

2. Medizinische Bildverarbeitungsvorrichtung (3) nach Anspruch 1, wobei der Bestimmungsteil ein Verfahren zum Ermitteln ausführt, ob jedes Ende des in dem Lokalbereich vorhandenen Randes tangential zu einem der Enden des Lokalbereichs verläuft, als Auswertung zum Bestimmen, ob der Lokalbereich durch mindestens einen Teil des durch das Randextraktionsteil extrahierten Randes geteilt wird.

3. Medizinische Bildverarbeitungsvorrichtung (3) zum Abschätzen eines dreidimensionalen Modells von einem lebenden Gewebe mit:
einem Dreidimensional-Koordinatendaten-Abschätzteil, das für jedes Pixel eines zweidimensionalen Eingangsbildes von einem lebenden Körpergewebe, welches von einer medizinischen Bildaufnahmevorrichtung (2) eingegeben wird, dreidimensionale Koordinatendaten abschätzt;
einem Dreidimensional-Modellgrößen-Bestimmungsteil zum Bestimmen einer Größe eines dreidimensionalen Modells in Voxeln auf der Grundlage der dreidimensionalen Koordinatendaten;
einem Lokalbereich-Einstellteil, das einen Lokalbereich auf ein interessierendes Voxel in den dreidimensionalen Koordinatendaten mittig einstellt;
einem Bestimmungsteil, das bestimmt, ob die Stückzahl der in dem Lokalbereich enthaltenen dreidimensionalen Koordinatendaten größer als ein vorgegebener Schwellenwert ist;
einem Formmerkmalswert-Berechnungsteil, das auf der Grundlage eines Ergebnisses der Bestimmung durch das Bestimmungsteil einen Formmerkmalswert beim interessierenden Voxel unter Verwendung der Teile der in dem Lokalbereich enthaltenen dreidimensionalen Koordinatendaten berechnet, wenn die Stückzahl der in dem Lokalbereich enthaltenen dreidimensionalen Koordinatendaten größer als der vorgegebene Schwellenwert ist; und
einem Formerhöhung-Ermittlungsteil, das eine erhöhte Form auf der Grundlage eines Ergebnisses der Berechnung durch das Formmerkmalswert-Berechnungsteil ermittelt.

4. Medizinische Bildverarbeitungsvorrichtung (3) nach Anspruch 3, die ferner aufweist:
ein Randextraktionsteil, das in dem zweidimensionalen Bild einen Rand extrahiert, wobei
das Lokalbereich-Einstellteil auf der Grundlage eines Ergebnisses der Randextraktion durch das Randextraktionsteil bestimmt, ob das interessierende Voxel ein Voxel ist, das zum Rand in dem zweidimensionalen Bild gehört, und abhängig von einem Ergebnis der Bestimmung eine Größe des Lokalbereichs ändert.

5. Medizinische Bildverarbeitungsvorrichtung (3) nach Anspruch 4, wobei der Lokalbereich-Einstellteil die Größe des Lokalbereichs auf eine erste Größe einstellt, wenn das interessierende Voxel kein zu dem Rand in dem zweidimensionalen Bild gehörendes Voxel ist, und die Größe des Lokalbereichs auf eine zweite Größe einstellt, die kleiner als die erste Größe ist, wenn das interessierende Voxel ein zum Rand in dem zweidimensionalen Bild gehörendes Voxel ist.

6. Medizinisches Bildverarbeitungsverfahren zum Abschätzen eines dreidimensionalen Modells von einem lebenden Gewebe mit:
einem Randextraktionsschritt zum Extrahieren eines Randbildes auf der Grundlage eines zweidimensionalen Eingangsbildes von einem lebenden Körpergewebe, das von einer medizinischen Bildaufnahmevorrichtung (2) eingegeben wird;
einem Dreidimensional-Koordinatendaten-Abschätzschritt zum Abschätzen dreidimensionaler Koordinatendaten für jedes Pixel des zweidimensionalen Eingangsbildes vom lebenden Körpergewebe;
einem Lokalbereich-Einstellschritt zum mittigen Einstellen eines Lokalbereichs auf ein interessierendes Pixel in dem zweidimensionalen Randbild;
einem Bestimmungsschritt zum Bestimmen, ob der Lokalbereich durch mindestens einen Teil des in dem Randextraktionsschritt extrahierten Randes in zwei Bereiche geteilt wird;
einem Kennzeichnungsschritt zum Kennzeichnen eines jeden der beiden Bereiche in dem Lokalbereich, der durch den Rand geteilt wird, oder zum Einstellen des gesamten Lokalbereichs als eine Kennzeichnung, wenn der Lokalbereich nicht durch den Rand in dem Lokalbereich geteilt wird;
einem Formmerkmalswert-Berechnungsschritt zum Berechnen eines Formmerkmalswerts des interessierenden Pixels unter Verwendung dreidimensionaler Koordinatendaten des Lokalbereichs, die zu einem Bereich mit derselben Kennzeichnung gehören wie die Kennzeichnung, zu der das interessierende Pixel in dem Lokalbereich gehört; und
einem Formerhöhung-Ermittlungsschritt zum Ermitteln einer erhöhten Form auf der Grundlage eines Ergebnisses der Berechnung in dem Formmerkmalswert-Berechnungsschritt.

7. Medizinisches Bildverarbeitungsverfahren nach Anspruch 6, wobei der Bestimmungsschritt das Ausführen eines Verfahrens zum Ermitteln umfasst, ob jedes Ende des in dem Lokalbereich vorhandenen Randes tangential zu einem der Enden des Lokalbereichs verläuft, als Auswertung zum Bestimmen, ob der Lokalbereich durch mindestens einen Teil des in dem Randextraktionsschritt extrahierten Randes geteilt wird.

8. Medizinisches Bildverarbeitungsverfahren zum Abschätzen eines dreidimensionalen Modells von einem lebenden Gewebe mit:
einem Dreidimensional-Koordinatendaten-Abschätzschritt zum Abschätzen dreidimensionaler Koordinatendaten für jedes Pixel eines zweidimensionalen Eingangsbildes von einem lebenden Körpergewebe, welches von einer medizinischen Bildaufnahmevorrichtung (2) eingegeben wird;
einem Dreidimensional-Modellgrößen-Bestimmungsschritt zum Bestimmen einer Größe eines dreidimensionalen Modells in Voxeln auf der Grundlage der dreidimensionalen Koordinatendaten;
einem Lokalbereich-Einstellschritt zum mittigen Einstellen eines Lokalbereichs auf ein interessierendes Voxel in den dreidimensionalen Koordinatendaten;
einem Bestimmungsschritt zum Bestimmen, ob die Stückzahl der in dem Lokalbereich enthaltenen dreidimensionalen Koordinatendaten größer als ein vorgegebener Schwellenwert ist;
einem Formmerkmalswert-Berechnungsschritt zum Berechnen eines Formmerkmalswerts beim interessierenden Voxel unter Verwendung der Teile der in dem Lokalbereich enthaltenen dreidimensionalen Koordinatendaten auf der Grundlage eines Ergebnisses der Bestimmung in dem Bestimmungsschritt, wenn die Stückzahl der in dem Lokalbereich enthaltenen dreidimensionalen Koordinatendaten größer als der vorgegebene Schwellenwert ist; und
einem Formerhöhung-Ermittlungsschritt zum Ermitteln einer erhöhten Form auf der Grundlage eines Ergebnisses der Berechnung in dem Formmerkmalswert-Berechnungsschritt.

9. Medizinisches Bildverarbeitungsverfahren nach Anspruch 8, das ferner aufweist:
einen Randextraktionsschritt zum Extrahieren eines Randes in dem zweidimensionalen Bild, wobei
der Lokalbereich-Einstellschritt auf der Grundlage eines Ergebnisses der Randextraktion in dem Randextraktionsschritt das Bestimmen aufweist, ob das interessierende Voxel ein Voxel ist, dem Rand in dem zweidimensionalen Bild entspricht, und das Ändern einer Größe des Lokalbereichs abhängig von einem Ergebnis der Bestimmung.

10. Medizinisches Bildverarbeitungsverfahren nach Anspruch 9, wobei der Lokalbereich-Einstellschritt das Einstellen der Größe des Lokalbereichs auf eine erste Größe aufweist, wenn das interessierende Voxel kein dem Rand in dem zweidimensionalen Bild entsprechendes Voxel ist, und das Einstellen der Größe des Lokalbereichs auf eine zweite Größe aufweist, die kleiner als die erste Größe ist, wenn das interessierende Voxel ein dem Rand in dem zweidimensionalen Bild entsprechendes Voxel ist.

## Revendications

1. Appareil de traitement d'images médicales (3) destiné à estimer un modèle en trois dimensions d'un tissu vivant, comprenant:
une partie d'extraction de bord qui extrait une image de bord sur la base d'une image d'entrée en deux dimensions d'un tissu de corps vivant, délivrée en entrée depuis un appareil de capture d'images médicales (2);
une partie d'estimation de données de coordonnées en trois dimensions qui estime les données de coordonnées en trois dimensions pour chaque pixel de l'image d'entrée en deux dimensions du tissu de corps vivant;
une partie de fixation de région locale qui fixe une région locale centrée sur un pixel d'intérêt dans l'image de bord en deux dimensions;
une partie de détermination qui détermine si la région locale est divisée en deux régions par au moins une partie du bord extrait par la partie d'extraction de bord;
une partie d'étiquetage destinée à étiqueter chacune desdites deux régions dans la région locale divisée par le bord ou à établir la région locale entière comme une étiquette si la région locale n'est pas divisée par le bord dans la région locale;
une partie de calcul de valeur de caractéristique de forme qui calcule une valeur de caractéristique de forme du pixel d'intérêt utilisant les données de coordonnées en trois dimensions de la région locale correspondant à une région avec la même étiquette que l'étiquette à laquelle le pixel d'intérêt appartient dans la région locale; et
une partie de détection de forme élevée qui détecte une forme élevée sur la base d'un résultat de calcul par la partie de calcul de valeur de caractéristique de forme.

2. Appareil de traitement d'images médicales (3) selon la revendication 1, dans lequel la partie de détermination réalise un processus consistant à détecter si chaque extrémité du bord présent dans la région locale est tangente par rapport à l'une quelconque des extrémités de la région locale en tant que traitement destiné à déterminer si la région locale est divisée par au moins une partie du bord extrait par la partie d'extraction de bord.

3. Appareil de traitement d'images médicales (3) destiné à estimer un modèle en trois dimensions d'un tissu vivant, comprenant:
une partie d'estimation de données de coordonnées en trois dimensions qui estime les données de coordonnées en trois dimensions pour chaque pixel d'une image d'entrée en deux dimensions d'un tissu de corps vivant, délivrée en entrée à partir d'un appareil de capture d'images médicales (2);
une partie de détermination de taille de modèle en trois dimensions destinée à déterminer une taille d'un modèle en trois dimensions en voxels, sur la base des données de coordonnées en trois dimensions;
une partie de fixation de région locale qui fixe une région locale centrée sur un voxel d'intérêt dans les données de coordonnées en trois dimensions;
une partie de détermination qui détermine si le nombre de parties des données de coordonnées en trois dimensions contenues dans la région locale est supérieur à une valeur de seuil prédéterminée;
une partie de calcul de valeur de caractéristique de forme qui calcule une valeur de caractéristique de forme au niveau du voxel d'intérêt en utilisant les parties des données de coordonnées en trois dimensions contenues dans la région locale si le nombre de parties de données de coordonnées en trois dimensions contenues dans la région locale est supérieur à la valeur de seuil prédéterminée, sur la base d'un résultat de détermination par la partie de détermination; et
une partie de détection de forme élevée qui détecte une forme élevée, sur la base d'un résultat de calcul par la partie de calcul de valeur de caractéristique de forme.

4. Appareil de traitement d'images médicales (3) selon la revendication 3, comprenant en outre:
une partie d'extraction de bord qui extrait un bord dans l'image en deux dimensions, dans lequel
la partie de fixation de région locale détermine, sur la base d'un résultat d'extraction de bord par la partie d'extraction de bord, si le voxel d'intérêt est un voxel correspondant au bord dans l'image en deux dimensions et change une taille de la région locale en fonction d'un résultat de la détermination.

5. Appareil de traitement d'images médicales (3) selon la revendication 4, dans lequel la partie de fixation de région locale fixe la taille de la région locale à une première taille si le voxel d'intérêt n'est pas un voxel correspondant au bord dans l'image en deux dimensions et fixe la taille de la région locale à une deuxième taille inférieure à la première taille si le voxel d'intérêt est un voxel correspondant au bord dans l'image en deux dimensions.

6. Procédé de traitement d'images médicales destiné à estimer un modèle en trois dimensions d'un tissu vivant, comprenant:
une étape d'extraction de bord destinée à extraire une image de bord sur la base d'une image d'entrée en deux dimensions d'un tissu de corps vivant, délivrée en entrée à partir d'un appareil de capture d'images médicales (2);
une étape d'estimation de données de coordonnées en trois dimensions consistant à estimer des données de coordonnées en trois dimensions pour chaque pixel de l'image d'entrée en deux dimensions du tissu de corps vivant;
une étape de fixation de région locale consistant à fixer une région locale centrée sur un pixel d'intérêt dans l'image de bord en deux dimensions;
une étape de détermination consistant à déterminer si la région locale est divisée en deux régions par au moins une partie du bord extrait dans l'étape d'extraction de bord;
une étape d'étiquetage consistant à étiqueter chacune desdites deux régions dans la région locale divisée par le bord ou à fixer la région locale entière comme une étiquette si la région locale n'est pas divisée par le bord dans la région locale;
une étape de calcul de valeur de caractéristique de forme consistant à calculer une valeur de caractéristique de forme du pixel d'intérêt en utilisant les données de coordonnées en trois dimensions de la région locale correspondant à une région avec la même étiquette que l'étiquette à laquelle le pixel d'intérêt appartient dans la région locale; et
une étape de détection de forme élevée consistant à détecter une forme élevée sur la base d'un résultat de calcul dans l'étape de calcul de valeur de caractéristique de forme.

7. Procédé de traitement d'images médicales selon la revendication 6, dans lequel l'étape de détermination comprend l'exécution d'un processus consistant à détecter si chaque extrémité du bord présent dans la région locale est tangente par rapport à l'une quelconque des extrémités de la région locale en tant que traitement destiné à déterminer si la région locale est divisée par au moins une partie du bord extrait dans l'étape d'extraction de bord.

8. Procédé de traitement d'images médicales destiné à estimer un modèle en trois dimensions d'un tissu vivant, comprenant:
une étape d'estimation de données de coordonnées en trois dimensions consistant à estimer des données de coordonnées en trois dimensions pour chaque pixel d'une image d'entrée en deux dimensions d'un tissu de corps vivant, délivrée en entrée à partir d'un appareil de capture d'images médicales (2);
une étape de détermination de taille de modèle en trois dimensions consistant à déterminer une taille d'un modèle en trois dimensions en voxels, sur la base des données de coordonnées en trois dimensions;
une étape de fixation de région locale consistant à fixer une région locale centrée sur un voxel d'intérêt dans les données de coordonnées en trois dimensions;
une étape de détermination consistant à déterminer si le nombre de parties de données de coordonnées en trois dimensions contenues dans la région locale est supérieur à une valeur de seuil prédéterminée;
une étape de calcul de valeur de caractéristique de forme consistant à calculer une valeur de caractéristique de forme au niveau du voxel d'intérêt en utilisant les parties des données de coordonnées en trois dimensions contenues dans la région locale si le nombre de parties de données de coordonnées en trois dimensions contenues dans la région locale est supérieur à la valeur de seuil prédéterminée, sur la base d'un résultat de détermination dans l'étape de détermination; et
une étape de détection de forme élevée consistant à détecter une forme élevée, sur la base d'un résultat de calcul dans l'étape de calcul de valeur de caractéristique de forme.

9. Procédé de traitement d'images médicales selon la revendication 8, comprenant en outre:
une étape d'extraction de bord consistant à extraire un bord dans l'image en deux dimensions, dans lequel
l'étape de fixation de région locale comprend l'étape consistant à déterminer, sur la base d'un résultat d'extraction de bord dans l'étape d'extraction de bord, si le voxel d'intérêt est un voxel correspondant au bord dans l'image en deux dimensions et à changer une taille de la région locale en fonction d'un résultat de la détermination.

10. Procédé de traitement d'images médicales selon la revendication 9, dans lequel l'étape de fixation de région locale comprend la fixation de la taille de la région locale à une première taille si le voxel d'intérêt n'est pas un voxel correspondant au bord dans l'image en deux dimensions et la fixation de la taille de la région locale à une deuxième taille inférieure à la première taille si le voxel d'intérêt et un voxel correspondant au bord dans l'image en deux dimensions.
